# EUROPÄISCHE PATENTANMELDUNG

(11) **EP 1 600 774 A1**
(43) Veröffentlichungstag der Anmeldung: **30.11.2005**
(21) Anmeldenummer: 04005940.4
(22) Anmeldetag: 12.03.2004
(51) Int. Cl.: G01N 33/50, G01N 33/68, G01N 33/58

(54) **Verfahren zur Identifizierung von Substanzen zur Beeinflussung der Aggregationen von Proteinen**

(71) Anmelder: Ludwig-Maximilians-Universität München, 80359 München (DE)
(72) Erfinder: Bertsch, Uwe, Dr., 86937 Scheuring (DE); Giese, Armin, Dr., 81477 München (DE); Kretzschmar, Hans, A., Prof., Dr., 82515 Wolfratshausen (DE); Bieschke, Jan, Dr., San Diego, CA 92109 (US)
(74) Vertreter: Schreiber, Christoph

(57) **Zusammenfassung**

Verfahren zur Identifizierung von Substanzen zur Beeinflussung von Aggregation mit folgenden Schritten:
a) Zusammenbringen mindestens eines Aggregates mit einer ersten detektierbaren Funktion und mindestens eines Monomers mit einer zweiten detektierbaren Funktion, wobei das mindestens eine Monomer eine Affinität für das mindestens eine Aggregat aufweist, in Gegenwart einer potenziell aggregationsbeeinflussenden Substanz.
b) Bestimmung eines Markierungsgrades der Aggregate, wobei der Markierungsgrad ein Maß für die Anzahl und das Verhältnis der gebundenen detektierbaren Funktionen ist.

## Beschreibung

Die vorliegende-Erfindung betrifft einen Screening-Assay zur Identifizierung von Substanzen zur Beeinflussung von Aggregationen.

Eine Reihe verschiedener Erkrankungen ist mit dem Auftreten von pathologischen Ablagerungen (Aggregaten), insbesondere Proteinaggregaten verbunden. So sind neurodegenerative Erkrankungen bekannt, bei denen beispielsweise im Gehirn der Betroffenen als amyloide Plaques bezeichnete Proteinablagerungen feststellbar sind. Zu solchen Erkrankungen gehören beispielsweise die Alzheimersche Erkrankung, bovine spongiforme Enzephalopathie (BSE), die Creutzfeldt-Jakob-Erkrankung (CJD), Kuru-Erkrankung, Scrapie. In jüngster Zeit ist insbesondere die BSE-Erkrankung ins Bewusstsein der Öffentlichkeit gerückt, unter anderem, weil BSE mit der Creutzfeldt-Jakob-Erkrankung beim Menschen in Zusammenhang gebracht wird. Bis heute ist nicht vollständig geklärt, aufgrund welcher Mechanismen die Proteinablagerungen in das Krankheitsgeschehen eingreifen. Bemerkenswert ist der von Prusiner beobachtete Zusammenhang zwischen Infektiösität und der Konzentration bestimmter Proteine, die beim Krankheitsgeschehen der Scrapie, einer neurodegenerativen Erkrankung von Schafen, eine Rolle spielen. Pathologische Proteinablagerungen treten nicht nur bei Erkrankungen des neuronalen Systems in Erscheinung, sondern werden auch in anderen Organen beobachtet, so zum Beispiel bei einer Erkrankung der Diabetes Typ II.

Eine Übersicht über Prionen-Krankheiten ist von D. Riesner in "Chemie in unserer Zeit" (1996), Seiten 66 bis 74 veröffentlicht worden. Speziell für die Alzheimersche Erkrankung ist das pathologische Bild relativ gut beschreiben. "Senile Plaques", die zum wesentlichen Teil aus aggregiertem Amyloid-β-Protein bestehen, sowie "Paired helical filaments", die aus abnormal verändertem tau-Protein aufgebaut sind, sind mit der Alzheimerschen Erkrankung eng verbunden.

Ansatzpunkt einer kausalen Therapie sollte es daher sein, die Aggregation zu verhindern oder zu verringern.

Die bisherigen Methoden zur Auffindung von Therapeutika für Amyloid-bedingte neurodegenerative Erkrankungen basieren entweder auf aufwendigen in vivo Testverfahren in kranken Tieren (oder gentechnisch erzeugten Tiermodellen) oder in (infizierten) Zellkulturen oder auf ebenfalls aufwendigen in vitro Verfahren, die häufig mit radioaktiver Markierung von Proteinen und langen Inkubationsphasen (1 bis 7 Tage) verbunden sind. Als Methode zur Auswertung der Wirksamkeit von Substanzen werden häufig Western-blot-Verfahren angewendet, die aus mehreren häufig stundenlangen Elektrophorese- und Inkubationsschritten bestehen und in der Regel nur eine beschränkte Anzahl von Proben in einem Durchgang (<30) zulassen.

Für in vivo Versuche werden vergleichsweise große Mengen der zu untersuchenden Substanzen benötigt, da Wirkkonzentrationen von bis zum 10 µM in einem Tier oder mehreren Millilitern einer Zellkulturschale oft über mehrere Tage oder Wochen aufrecht erhalten werden müssen.

Aufgabe der Erfindung ist die zur Verfügungsstellung eines Verfahrens zur Identifizierung von Substanzen zur Beeinflussung von Aggregationen, das die oben genannten Nachteile überwindet und insbesondere einen hohen Durchsatz bei kleinen Substanzmengen und ein weitgehend automatisierbares Verfahren ermöglicht.

Gelöst wird die Aufgabe durch ein Verfahren mit den Merkmalen des Patentanspruchs 1.

Das erfindungsgemäße Verfahren zur Identifikation von Substanzen zur Beeinflussung von Aggregationen weist folgende Schritte auf:
a) Zusammenbringen mindestens eines Aggregates mit einer ersten detektierbaren Funktion und mindestens eines Monomers mit einer zweiten detektierbaren Funktion, wobei das mindestens eine Monomer eine Affinität für das mindestens eine Aggregat aufweist, in Gegenwart einer potenziell aggregationsbeeinflussenden Substanz.
b) Bestimmung eines Markierungsgrades der Aggregate.

Der Markierungsgrad ist ein Maß für die Anzahl und das Verhältnis der gebundenen detektierbaren Funktionen.

Erfindungsgemäß wird ein Aggregat, das eine detektierbare Funktion aufweist, zusammen mit einem Monomer mit einer zweiten davon unterscheidbaren detektierbaren Funktion, inkubiert. Dabei muss das Monomer in der Lage sein, an das Aggregat zu binden. Die Inkubation erfolgt in Gegenwart einer potentiellen aggregationsbeeinflussenden Substanz. Die Hemmung der Bindung des Monomers an das Aggregat wird dadurch bestimmt, dass die Aggregate sowohl im Hinblick auf die erste als auch auf die zweite detektierbare Funktion vermessen werden.

Aggregate im Sinne dieser Anmeldung bezeichnet eine Aneinanderlagerung von Strukturen aus im wesentlichen gleichen Bausteinen und Bindungsmöglichkeiten für weitere Einheiten. In einer Ausführungsform handelt es sich um pathologische Proteinaggregate. Typische Proteinaggregate bestehen aus den Komponenten des Prion-Proteins, APP, tau, α-Synuclein oder Proteinen mit einer Polyglutaminsequenz wie Huntingtin, Fragmenten oder Derivaten dieser Proteine.

Alternativ können die Aggregate auch aus anderen Bausteinen, insbesondere Nukleinsäuren, Lipide, Polysaccharide, vesikulären Systemen, oder Nanoelementen bestehen.

Die erfindungsgemäß eingesetzten Aggregate sind in der Lage, die als Monomer bezeichneten Einheiten zu binden.

Monomer bezeichnet eine Struktur, die in der Lage ist, an Aggregate zu binden und Bestandteil von Komplexen aus mehr als einem monomeren Baustein sein kann.

In einer bevorzugten Ausführungsform sind das Monomer und das Aggregat von der chemischen Struktur her ähnlich, d.h. das Aggregat setzt sich aus Bestandteilen zusammen, die entweder dem Monomer entsprechen oder eine ähnliche biologische Funktion haben aber beispielsweise ein Fragment, Derivat, etc. sind, beispielsweise mit einer geringfügig abweichenden chemischen Struktur.

Eine detektierbare Funktion im Sinne dieser Anmeldung ist eine Möglichkeit, die Funktion spezifisch in dem Verfahren nachweisen zu können. Typisch detektierbare Funktionen sind beispielsweise radioaktive Markierungen, Farbstoffmarkierungen, wie Fluoreszenzmarkierung. Dabei kann die detektierbare Funktion sowohl direkt an das Aggregat oder das Monomer gebunden sein. Sie kann jedoch auch indirekt gebunden sein (Sekundärmarkierung), beispielsweise indem Aggregate oder Monomere mit einem Antikörper gebunden werden, der wiederum selbst eine detektierbare Funktion wie ein Fluoreszenzmolekül, etc. aufweist.

Die erste und zweite detektierbare Funktion sind erfindungsgemäß unterscheidbar.

Partikel im Sinne dieser Anmeldung sind Einheiten, die detektierbar sind, insbesondere die Aggregate, Monomere oder Komplexe von Aggregaten mit einem oder mehreren Monomeren gegebenenfalls zusammen mit weiteren Molekülen, beispielsweise Antikörpern.

In einer besonders bevorzugten Ausführungsform wird die erste detektierbare Funktion über ein Bindungsmolekül an das Aggregat gebunden, wobei das Bindungsmolekül eine hohe Affinität für Aggregate und eine niedrige Affinität für Monomere aufweist. Die Begriffe "hoch" und "niedrig" sind hier relativ zueinander zu verstehen, d.h. die Affinität für die Aggregate muss höher sein als für die Monomere. Die absolute Affinität ist von geringerer Bedeutung. Typischerweise unterscheidet sich die Affinität für das Aggregat (K_{D}-Wert) mindestens um den Faktor 10 von der Affinität für das Monomer.

Bevorzugte Bindungsmoleküle sind beispielsweise Antikörper, Fragmente von Antikörpern oder rekombinante Moleküle mit der Bindungsfunktion eines Antikörpers wie scFv-Fragmente.

Besonders geeignete detektierbare Funktionen sind Fluoreszenzmoleküle. Dabei kann nur die erste, nur die zweite oder beide detektierbare Funktionen in Form von Fluoreszenzmolekülen vorliegen.

Erfindungsgemäß ist es besonders vorteilhaft, wenn der Anteil an Aggregaten auf Basis einzelner Partikel bestimmt wird, d.h., dass durch die Messung sicher gestellt wird, dass eine Vielzahl von Aggregaten jeweils einzeln gemessen werden können.

In vorteilhafter Weise kann auch die Anzahl und das Verhältnis aller detektierbaren Funktionen gemessen werden, insbesondere aller detektierbaren Funktionen, die an Partikeln gebunden sind. Eine hierfür besonders geeignete Messmethode ist das Verfahren, dass in der WO 01/23894 ausführlich beschrieben ist. Auf diese Anmeldung wird ausdrücklich Bezug genommen. Einzelheiten hierzu werden noch erläutert.

Das erfindungsgemäße Verfahren wird in einer besonders bevorzugten Ausführungsform zur Wirkstofffindung bei Proteinaggregationskrankheiten eingesetzt. Wobei unter Proteinaggregationskrankheiten sowohl Krankheiten verstanden werden, bei denen die Aggregation primär ist (z. B. Prionenkrankheiten), als auch Krankheiten, bei denen die Aggregation sekundär ist, aber zum Gewebeschaden beiträgt. Hierbei ist das Aggregat ein Proteinaggregat und das Monomer ein Proteinmonomer und die aggregationsbeeinflussende Substanz ein potentieller Wirkstoff zur Behandlung einer Proteinaggregationskrankheit.

Das Proteinaggregat kann dann ein Multimer des Proteinmonomers sein. Diese können sich jedoch auch strukturell unterscheiden, solange noch eine Affinität zwischen Proteinaggregat und Proteinmonomer besteht.

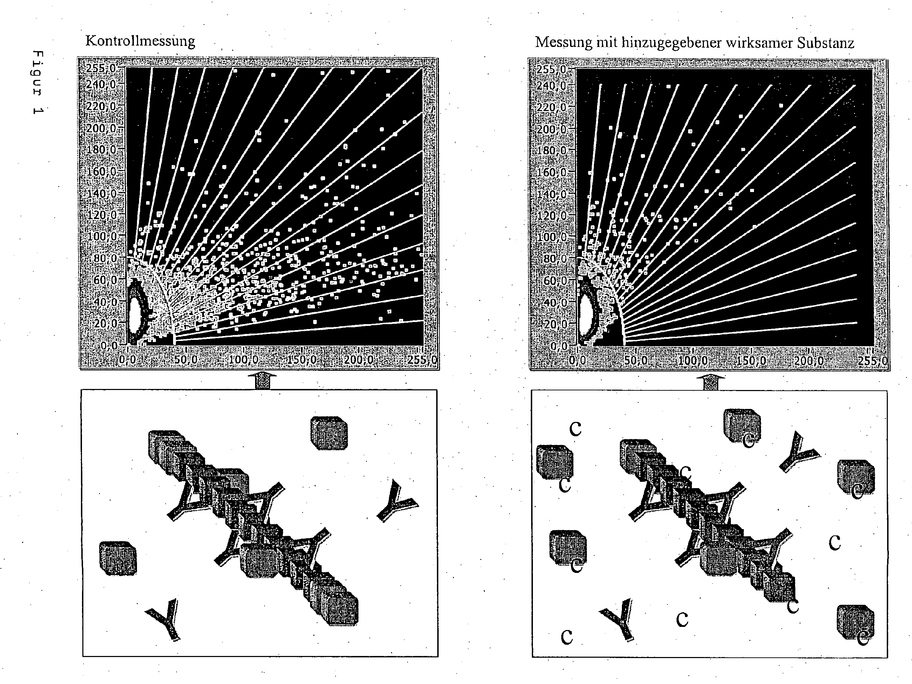

Das erfindungsgemäße Verfahren wird an der Figur 1 erläutert. In einer Kontrollmessung werden Aggregate (in der Figur als verbundene dunkle Einheiten dargestellt) zusammen mit Monomeren (helle Einheiten) inkubiert. Dabei tragen die hellen Einheiten eine detektierbare Funktion, beispielsweise einen Fluoreszenzfarbstoff. Die Proteinaggregate werden durch Zugabe eines Antikörpers, der spezifisch für die Aggregate ist, jedoch praktisch nicht an die Monomere bindet, mit einem zweiten Farbstoff markiert. Es stellt sich dann in Abhängigkeit von den Konzentrationsverhältnissen ein Verhältnis von Partikeln ein, die nur die erste Markierung tragen oder nur die zweite Markierung, oder Partikel, die beide Markierungen in einem gewissen Verhältnis tragen. Zur Auswertung wird, wie in der Figur 1 dargestellt, beispielsweise der Umfang der Markierung einzelner Partikel gegeneinander auf zwei Achsen aufgetragen.

In der rechten Grafik ist eine potentielle Wirk-Substanz "C" zugegeben. Durch Bindung an das Monomer verhindert sie teilweise eine Bindung an das Aggregat. Dies führt zu einer Veränderung der Markierung an den Aggregaten und damit einhergehend zu einer entsprechenden Verschiebung im Fluoreszenzmuster.

Das erfindungsgemäße Verfahren, das oben beschrieben ist, eignet sich zur Wirkstofffindung, aber auch als allgemeiner Assay zur Identifikation von Substanzen, die eine Aggregation beeinflussen.

Das erfindungsgemäße Verfahren eignet sich auch zur Analyse beliebiger anderer Aggregate, beispielsweise von Nukleinsäuren, Vesikeln oder Nanoelementen. Bei Nanoelementen handelt es sich um organische oder anorganische Moleküle, die größere Strukturen in einem Selbstorganisationsprozess bilden. Nanoelemente sind beispielsweise Fullerene oder Molekül-/Atomcluster (rein oder gemischt hergestellt).

Insbesondere können auch Aggregate vermessen werden, bei denen die Aggregate und Monomere aus verschiedenen Stoffklassen stammen, beispielweise Nukleinsäuren + Protein, wie es bei Ribosomen oder hisfonverpackter DNA der Fall ist, beliebige vesikuläre Mizelläre oder Stützstrukturen oder die Bindung von Substanzen an Polysaccharide.

Die Messung der Aggregation kann grundsätzlich sowohl in zellfreien Systemen in einer Zelle als auch im Überstand einer Zellkultur erfolgen.

Als Messverfahren eignen sich unterschiedliche Techniken. Eine Möglichkeit sind bildgebende Verfahren, insbesondere im Falle von Fluoreszenz oder sonstigen farbstoffmarkierten Partikeln, zum Beispiel ein Opera® System der Firma Evotec Technologies.

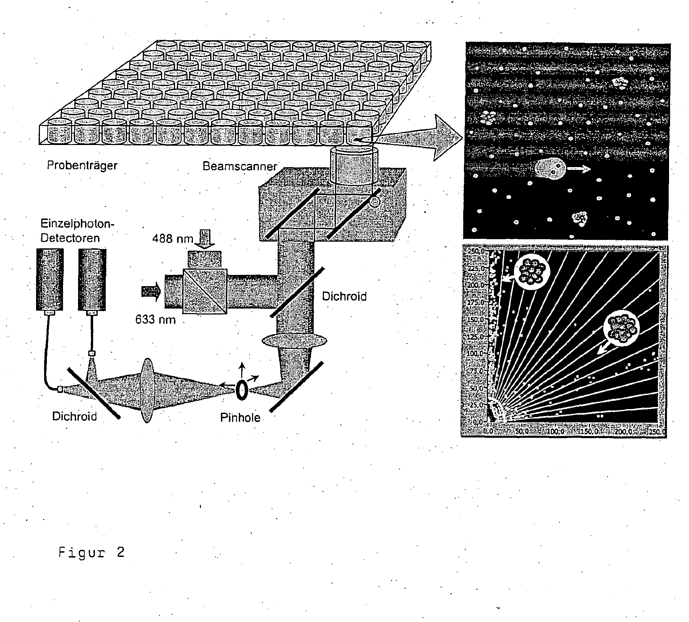

Ein besonders geeignetes Verfahren ist in der WO 01/23894 A1 beschrieben und wird in einer vorteilhaften Ausgestaltung als SIFT (Scanning for Intensely Fluorescent Targets) bezeichnet wird. Sie beruht auf einer zeitaufgelösten Intensitätsanalyse eines Fluoreszenzsignals in einem offenen Volumenelement, das durch eine konfokale Figur eines oder mehrere in einem Fokus gebündelter Anregungslaser definiert wird. Hierdurch erfolgt eine Quantifizierung des partikelbedingten Signalanteils, vorzugsweise durch Analyse der Intensitätsverteilung eines gemessen Detektionssignals, beispielsweise eines Fluoreszenzsignals, in sukzessiven Zeitfenstern. Diese Zeitfenster werden als BINs bezeichnet. Typische Detektionszeiten liegen im Bereich von µ- bis Millisekunden und können konstante und variable Längen aufweisen, wodurch das sehr intensive Signal der mehrfach markierten Partikel vom Hintergrundsignal abgetrennt werden kann. Das Scannen der Probe kann durch eine im wesentlichen konstante Relativbewegung zwischen Probe und Messvolumen unterstützt werden. Hierdurch wird das untersuchte Volumen und dabei die Messsensitivität erhöht. Hiermit ergeben sich insbesondere bei langsam diffundierenden Partikeln Vorteile, da die Aufenthaltsdauer nicht mehr durch die Diffusionszeit, sondern durch die Scanngeschwindigkeit bestimmt wird. Ein typischer Aufbau ist in Figur 2 dargestellt.

Das Verfahren ist auch in Bieschke et al. PNAS (2000), 5468 bis 5473 beschrieben.

Das erfindungsgemäße Verfahren wird durch das folgende Beispiel weiter erläutert.

### Beispiel 1

### Assay für Anti-Prionen-Substanz

Auf Basis der SIFT-Technik wird mit Hilfe eines Fluoreszenzkorrelationsspektroskops die Assoziationsreaktion von rekombinant hergestelltem Maus-PrP (Aminosäuren 23-231) und (hier nach der Methode von Safar et al., Nat Med. 1998 Oct;4(10):1157-65.) aus Hirngewebe von CJD-Patienten präparierten PrP-Sc Aggregaten untersucht. Für diese Assoziationsreaktion wird das rekombinante Maus-PrP mit einem Fluoreszenzfarbstoff (hier: Alexa488) kovalent an Lysinresten markiert. Die PrP-Sc Aggregate werden hingegen durch den Zusatz eines mit einem zweiten Fluoreszenzfarbstoff (hier: Alexa647) markierten monoklonalen Antikörpers (hier: L42 [Vorberg et al., Virology. 1999 Mar 1;255(1):26-31]) gegen menschliches PrP markiert. Der verwendete Antikörper bindet an das menschliche PrP-Sc, nicht aber an das zugefügte rekombinante Maus-PrP.

Die PrP-Sc Aggregate, die aufgrund der Bindung vieler monoklonaler Antikörper eine große Anzahl von Fluorophoren tragen und damit eine starke Fluoreszenz aufweisen, lagern bei den vorgegebenen Bedingungen (hier: 20mM Kaliumphosphat-Puffer pH 6,0; 0,1% Nonidet-P40) das rekombinante Maus-PrP ebenfalls in großer Zahl an. Es entstehen so Aggregate aus menschlichem PrP-Sc und fluoreszenz-markiertem Antikörper sowie fluoreszenz-markiertem Maus-PrP, die daher hohe Fluoreszenzintensitäten für beide Farbstoffe aufweisen. Solche hochintens zweifarbig fluoreszierenden Aggregate können mit der SIFT-Technik in der verwendeten FCS-Apparatur quantitativ erfasst und von eventuell auftretenden Aggregaten der Antikörper bzw. des Maus-PrPs alleine unterschieden werden. Bei der erfindungsgemäß bevorzugt verwendeten 2D-SIFT Auswertung werden die beobachteten Messzeitpunkte entsprechend der jeweils gemessenen Fluoreszenzintensität der beiden Farbstoffe in einem zweidimensionalen Intensitätshistogramm gegeneinander aufgetragen und hieraus ein Mass für die Menge der Aggregate mit bestimmten Markierungsverhältnissen der beiden detektierten Sondenspezies ermittelt. Die Quantifikation erfolgt dabei typischerweise automatisiert durch die Aufsummierung aller oberhalb eines Schwellenwertes gelegenen Messzeitpunkte, mit ähnlichem Farbintensitätsverhältnis, die entsprechend ihrer Zugehörigkeit zu einem Winkelbereich des 2-SIFT-Diagramms sektorenweise summiert werden.

Zu dieser Assoziationsreaktion zwischen PrP-Sc und Maus-PrP werden bei diesem Screening-Verfahren für Anti-Prion-Medikamente nun Substanzen zugegeben (eine typische hierbei verwendete Endkonzentration liegt bei ca. 10 µM) und die Verhinderung der Anlagerung des Maus-PrP an die PrP-Sc Aggregate analysiert. Substanzen, die diese Anlagerung verhindern, bewirken in der 2D-SIFT Auswertung eine Verschiebung des Verhältnisses der Fluoreszenzintensitäten der beiden Farbstoffe für die detektierten Aggregate hin zur Farbe des Antikörpers. Im Extremfall einer vollständigen Unterdrückung der Maus-PrP Bindung an die Aggregate, leuchten diese nur noch in der Farbe des Antikörpers.

Das erfindungsgemäße Verfahren ist weitgehend automatisierbar und erlaubt den Nachweis der Wirksamkeit einer Substanz mit einer kurzen Messzeit (typischerweise weniger als 75 s pro Substanz) und einer direkt online ausgeführten quantitativen Auswertung des Ergebnisses. Ein besonderer Vorteil des Verfahrens liegt auch darin, dass es mit extrem kleinen Substanzmengen arbeitet. So werden für eine Messung einer Substanz in einem 20 µl großen Volumen mit einer Endkonzentration von 10 µM nur 0,2 nmol der Substanz benötigt.

Als vorbereitende Arbeiten für das Verfahren sind typischerweise lediglich die Aufreinigung von Proteinaggregaten und die Fluoreszenzmarkierung von Antikörper und bakteriell hergestelltem und gereinigtem Protein-Monomer nicht vollständig automatisierbar. Diese Vorarbeiten werden jedoch für eine große Anzahl von Einzelmessungen in einem Durchgang ausgeführt, so dass die dafür benötigte Arbeitszeit für die Einzelmessung kaum ins Gewicht fällt. Der eigentliche Screeningassay kann dann als homogener Assay nach dem Prinzip des "Mix and Measure" ohne weitere Trennschritte direkt gemessen werden.

### Beispiel 2

### Validierung des Verfahrens

Das Verfahren wurde mit einer Modellsubstanz validiert, von der bekannt ist, dass sie die Bildung von PrP-Sc während des infektiösen Prozesses in vivo beeinflusst.

Als potentielles Prion-Therapeutikum wurde das polykationische Lipid 2,3-dioleyloxy-N-[2(sperminecarboxamido)ethyl]-N,N-dimethyl-1-propanaminium trifluoroacetate (DOSPA) verwendet. Es wurde gefunden, dass es die Assoziation von fluoreszenzgelabeltem rekombinantem PrP an Prionaggregate bei geringen mikromolaren Konzentrationen hemmt. In Kontrollen mit anderen Lipiden wie Dioleoyl-L-phosphatidylethanolamine (DOPE) wurde, wie erwartet, kein Einfluss auf die Anlagerung gefunden.

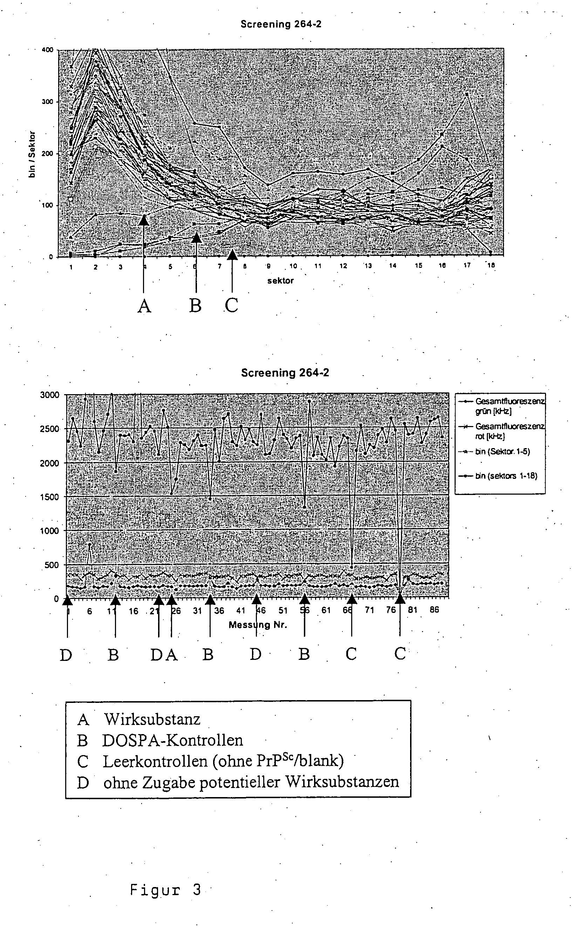

Figur 3 zeigt ein Screening-Assay für 80 Substanzen und acht Kontrollen in einem Mikrotiterplattenformat.

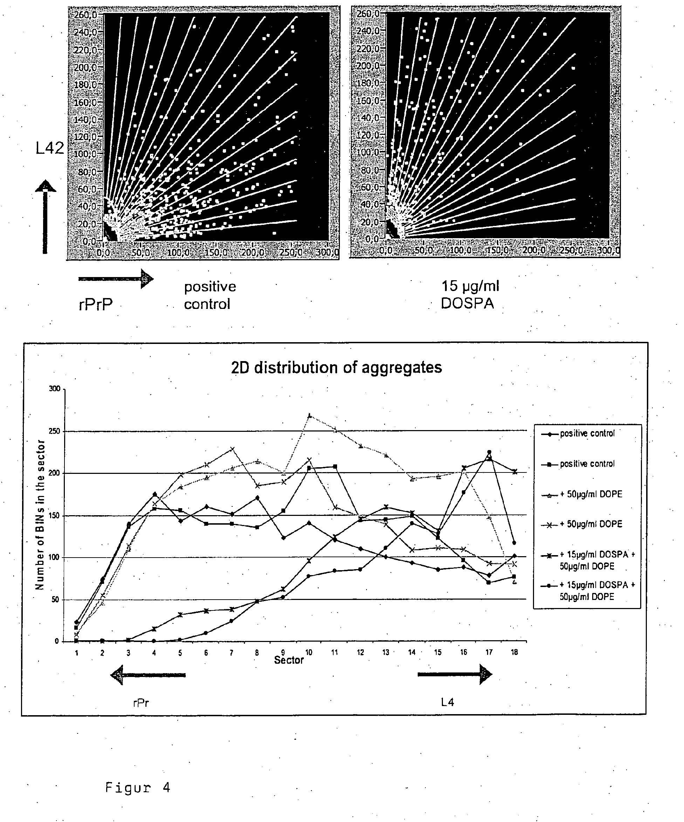

Figur 4 zeigt die Ergebnisse der Untersuchungen mit DOSPA. Hierzu wurden die 2D-Histogramme der Fluoreszenzintensität quantitativ analysiert durch Auszählung der Anzahl von BINs in jedem Sektor. Hierbei wird bei der Auswertung deutlich, dass sich die Anzahl der BINs in den Sektoren 1 bis 9 verringert.

## Patentansprüche

1. Verfahren zur Identifizierung von Substanzen zur Beeinflussung von Aggregation
mit folgenden Schritten:
a) Zusammenbringen mindestens eines Aggregates mit einer ersten detektierbaren Funktion und mindestens eines Monomers mit einer zweiten detektierbaren Funktion, wobei das mindestens eine Monomer eine Affinität für das mindestens eine Aggregat aufweist, in Gegenwart einer potenziell aggregationsbeeinflussenden Substanz.
b) Bestimmung eines Markierungsgrades der Aggregate, wobei der Markierungsgrad ein Maß für die Anzahl und das Verhältnis der gebundenen detektierbaren Funktionen ist.

2. Verfahren nach Anspruch 1, **dadurch gekennzeichnet, dass** die erste detektierbare Funktion an ein Bindungsmolekül gebunden ist, wobei das Bindungsmolekül eine hohe Affinität für das mindestens eine Aggregat und eine niedrige Affinität für das mindestens eine Monomer aufweist.

3. Verfahren nach Anspruch 2, **dadurch gekennzeichnet, dass** das Bindungsmolekül ein Antikörper, ein Fragment eines Antikörpers oder ein rekombinantes Molekül mit der Bindungsfunktion eines Antikörpers ist.

4. Verfahren nach mindestens einem der Ansprüche 1 bis 3, **dadurch gekennzeichnet, dass** die erste und/oder zweite detektierbare Funktion ein Fluoreszenzmolekül ist.

5. Verfahren nach mindestens einem der Ansprüche 1 bis 4, **dadurch gekennzeichnet, dass** der Markierungsgrad an Aggregaten auf Basis einzelner Partikel bestimmt wird.

6. Verfahren nach mindestens einem der Ansprüche 1 bis 5, **dadurch gekennzeichnet, dass** die Anzahl und das Verhältnis aller detekierbaren Funktionen gemessen wird.

7. Verfahren nach mindestens einem der Ansprüche 1 bis 6, **dadurch gekennzeichnet, dass** die Messung mit Hilfe der SIFT-Technik erfolgt.

8. Verfahren nach mindestens einem der Ansprüche 1 bis 7, **dadurch gekennzeichnet, dass** das Aggregat ausgewählt ist aus der Gruppe bestehend aus Proteinen, Nukleinsäuren, Lipiden, Polysacchariden, vesikulären Systemen und Nanoelementen.

9. Verfahren nach mindestens einem der Ansprüche 1 bis 8, **dadurch gekennzeichnet, dass** das Monomer ausgewählt ist aus der Gruppe bestehend aus Proteinen, Nukleinsäuren, Lipiden, Polysacchariden, vesikulären Systemen und Nanoelementen.

10. Verfahren nach mindestens einem der Ansprüche 1 bis 9, **dadurch gekennzeichnet, dass** das Aggregat ein Multimer des Monomers ist.

11. Kit enthaltend mindestens ein Aggregat mit einer ersten detektierbaren Funktion und mindestens ein Monomer mit einer zweiten detektierbaren Funktion.
